**Europäisches Patentamt**

**European Patent Office**

**Office européen des brevets**

(19)

(11) Publication number: **0 042 309**
**A1**

(12)

# EUROPEAN PATENT APPLICATION

(21) Application number: **81302737.2**

(22) Date of filing: **17.06.81**

(51) Int. Cl.³: **C 07 C 1/04,** C 07 C 29/15, B 01 J 31/12

(30) Priority: **18.06.80 US 160601**

(43) Date of publication of application: **23.12.81**
**Bulletin 81/51**

(84) Designated Contracting States: **BE DE FR GB IT NL**

(71) Applicant: **Ozyagcilar, Mehmet N.,**
**Apartment 807 3145 Queen Frederica Drive, Mississauga**
**Ontario L4Y3A7 (CA)**

(72) Inventor: **Ozyagcilar, Mehmet N.,**
**Apartment 807 3145 Queen Frederica Drive, Mississauga**
**Ontario L4Y3A7 (CA)**

(74) Representative: **Ritter, Stephen David et al, Mathys &**
**Squire 10 Fleet Street, London EC4Y 1AY (GB)**

(54) **Method for the coproduction of hydrocarbons and alcohols.**

(57) Methods for the synthesis of hydrocarbons and alcohols by contacting carbon monoxide and hydrogen over novel catalyst compositions are disclosed. The catalyst compostions are intermetallic compounds having a first and second phase, said first phase being selected from:

(1) a single metal selected from V and Nb,

(2) a single phase binary alloy selected from TiCo, TiCr, TiCu and TiMn and the binary Laves phase compounds $TiCr_2$, $ZrNi_2$, $ZrMo_2$, $ZrFe_2$, $ZrCo_2$, $ZrMn_2$, $TiCo_2$, $ZrCu_2$ and $ZrCr_2$, and

(3) single phase ternary or quaternary alloys selected from

(a) $TiFe_xM_yN_z$ where M and N are any metal that can substitute for Fe such that the resulting alloy is a single phase alloy, but not Ni or Ru, and where X is a number less than 1.0, y and z are each numbers less than 1.0 or either, but not both, may be 0, and x and y and z is approximately 1.0;

(b) $TiA_xM_yN_z$ where A is Co, Cr, Mn or Cu, M and N are any metal that can substitute for Co, Cr, Mn or Cu such that the resulting alloy is a single phase alloy, and where x is a number less than 1.0, y and z are each numbers less than 1.0 or either, but not both, may be 0, and x and y and z is approximately 1.0; and

(c) ternary or higher Laves phase compounds based on Ti and/or Zr, and said second phase is free titanium hydride or zirconium hydride.

0042309

## METHOD FOR THE COPRODUCTION OF HYDROCARBONS AND ALCOHOLS

This invention relates to the production of hydrocarbons and alcohols from mixtures of hydrogen and carbon monoxide, especially mixtures largely composed of hydrogen and carbon monoxide that are derived from the gasification of liquid and solid fuels, utilizing new catalysts and to novel processes based on the use of these new catalysts.

Prior art methods for the production of hydrocarbons such as methane and ethane have employed carbon monoxide and hydrogen over nickel catalysts of various types. The cost of nickel catalyst is quite high, both because of raw material costs and because of difficulties experienced in the manufacturing process, the latter requiring that the nickel be supported on some type of inert base. Moreover, the attrition of nickel catalyst is high because the processes that have been used require relatively high temperatures which in turn cause a sintering type of breakdown that rapidly decreases the activity of the catalyst as a function of time.

Nickel catalysts are also sensitive to poisoning by a number of impurities usually found in the hydrogen and carbon oxide reactants. For example, hydrogen sulfide has been found to poison nickel catalysts even in very small concentrations by forming nickel sulfide. The thermodynamics of the system is such that sulfur poisoning can be reversed by raising the temperature or increasing the hydrogen to hydrogen sulfide ratio in the feed or both. However, the use of higher operating temperatures with nickel catalysts is restricted by a sintering problem. Also the purity of the feed gas is inherently limited as a practical matter by the costs of purification.

The reaction of carbon monoxide with hydrogen in the presence of nickel catalyst requires temperatures in the range of $300^{\circ}$ to $500^{\circ}$C. to produce significant reaction rates. Temperatures within this range cause relatively rapid deterioration of the nickel catalyst. In addition, very little ethane can be produced with nickel catalysts since the ethane reaction is favored only at lower temperatures. Furthermore, practically no alcohol formation is observed over nickel catalysts. In general, the formation of alcohols is believed to follow a mechanism different from that prevailing in the synthesis of hydrocarbons. Heretofore a different catalyst, namely, zinc oxide, was required for the synthesis of methanol.

A further problem restricting the use of prior art catalysts is carbonyl formation. The carbonyls of metals like nickel, ruthenium and iron are extremely toxic compounds. They also have very low boiling temperatures and thus would be present in a vapor phase at the temperatures required for the synthesis of methane, ethane and higher carbon compounds. Carbonyl formation causes depletion of the catalyst as well as posing a severe health and safety problem. Such problems with prior art catalysts can be avoided only by carefully controlling the operating temperature, pressure, carbon oxide to hydrogen ratio and other operation parameters and conditions.

Even at the elevated temperatures indicated, the reaction rates with nickel and other known catalysts are relatively slow and require a high residence time in the reactor vessel, which in turn results in a relatively slow production rate for the final product desired.

In my prior U.S. Patent Specification No. 4,139,551, a process is disclosed for producing methane and ethane from hydrogen and oxides of carbon that avoids a number of the foregoing disadvantages. In this prior process methane and ethane are produced from mixtures of hydrogen and one or more oxides of carbon by passing the mixture over an iron/titanium catalyst wherein the atomic ratio of titanium to iron is preferably about 1.1:1. By using the iron/titanium catalyst, lower temperatures and pressures can be used than are required with the nickel catalyst and the formation of toxic carbonyl compounds is avoided. Also higher reaction rates can be achieved.

It is a general object of the invention to provide novel and improved processes (1) for the manufacture of hydrocarbons and alcohols from oxides of carbon and hydrogen, (2) for the co-production of hydrocarbons and alcohols, (3) for the production of alcohols at lower temperatures and pressures than heretofore possible, and/or (4) for the removal of oxides of carbon from chemical process streams where their presence is undesirable.

The process of the invention may further be utilized (1) to increase the heating value of gas initially obtained from gasification of coal, residua, oil shale, tar sands and similar carbonaceous substances by converting the carbon monoxide present in such a gas to methane, ethane and higher hydrocarbons as well as alcohols and (2) to provide a commercially feasible process for co-production of substitute natural gas, LPG, gasoline, liquid hydrocarbons, and alcohols that can be mixed with gasoline in an integrated plant from a feed obtained by the gasification of coal, residua, oil shale, tar sands and similar carbonaceous materials through the use of known gasification technology.

The objects and advantages of the present invention are achieved in general by contacting carbon monoxide and hydrogen in synthesis proportions under synthesis conditions over a catalyst to form hydrocarbons and alcohols.

The catalysts used in the invention are inter-metallic compounds of a specific composition. The catalysts consist essentially of a first phase (hereinafter referred to as phase A) defined more particularly hereinafter and a second phase (hereinafter referred to as phase B) of free titanium hydride or zirconium hydride. The free titanium hydride or zirconium hydride phase is usually formed under the conditions of catalyst activation and is maintained under the conditions of the process for production of hydrocarbons and alcohols, as further described below.

Phase A may be a single metal or may be a single phase alloy comprising a binary, ternary or quaternary intermetallic compound. Where phase A is a single metal, it is selected from the group consisting of V and Nb.

Where phase A is a single phase binary alloy, the binary alloy is selected from TiCo, TiCr, TiCu or TiMn, or may be selected from binary Laves phase compounds $TiCr_2$, $ZrNi_2$, $ZrMo_2$, $ZrFe_2$, $ZrCo_2$, $ZrMn_2$, $TiCo_2$, $ZrCu_2$ or $ZrCr_2$.

Where phase A is a single phase ternary or quaternary alloy, it is selected from

(a) $TiFe_xM_yN_z$ where M and N are any metal that can substitute for Fe such that the resulting alloy is a single phase alloy, but not Ni or Ru, and where x is a number less than 1.0, y and z are each numbers less than 1.0 or either, but not both, may be 0, and x and y and z is approximately 1.0;

(b) $TiA_xM_yN_z$ where A is Co, Cr, Mn or Cu, M and N are any metal that can substitute for Co, Cr, Mn or Cu such that the resulting alloy is a single phase alloy, and where x is a number less than 1.0, y and z are each numbers less than 1.0 or either, but not both, may be 0, and x and y and z is approximately 1.0; and

(c) ternary or higher Laves phase compounds based on Ti and/or Zr. Among the ternary or higher Laves phase compounds are:

(i) $TiB_xM_yN_z$ where B is Cr or Co, M and N are any metals that can substitute for Cr or Co such that the resulting alloy is a single phase alloy, and where x is a number less than 2.0, y and z are each numbers less than 2.0 or either, but not both, may be 0, and x and y and z is approximately 2.0 and

(ii) $ZrC_xM_yN_z$ where C is Ni, Mo, Fe, Co, Mn, Cr or Cu, M and N are any metals that can substitute for Ni, Mo, Fe, Co, Mn, Cr or Cu such that the resulting alloy is a single phase alloy, and where x is a number less than 2.0, y and z are each numbers less than 2.0 or either, but not both, may be 0, and x and y and z is approximately 2.0.

Among the preferred species of phase A are ternary and quaternary alloys of the formula $TiFe_xM_yN_z$ as defined above where M and N are Co, Cr, Mn, Mo, Va and Cu.

As understood by those skilled in the art, the intermetallic compounds have a range of compositions in which they are homogeneous and any such homogeneous compositions may be satisfactorily employed as Phase A.

Phase A of the catalysts used in the invention are formed by melting processes known in the art requiring elevated temperatures typically above $1500^{\circ}C$. Where iron is a component of Phase A, it can be commercial grade iron although the purity of electrolytic iron is preferred.

The catalyst composition, consisting essentially of Phases A and B, can also be produced by methods known in the art. Where Phase B is titanium hydride, it is a non-stoichiometric hydride containing slightly less hydrogen than the stoichiometric dihydride and may, for example, have the approximate formula $TiH_{1.98}$. This latter titanium hydride is a relatively stable compound which remains as such throughout the production process of the invention and is continually activated (decontaminated) as long as it is in contact with significant amounts of hydrogen in the synthesis process. While not wishing to be bound by any theory, it is believed that the catalytically active component is Phase B, hydrided titanium or zirconium. Further information on the properties of titanium hydride is found in W.M. Mueller, et al., Metal Hydrides, Academic Press, New York, New York 1978.

As pointed out more fully below, before being used in the synthesis process, the catalyst is desirably activated by alternate hydriding and dehydriding steps to cause a partial physical disintegration of the catalyst and thereby increase its surface area. Also it has been found advantageous when employing the present catalyst in the synthesis of hydrocarbons and alcohols to carry out the process using a stoichiometric excess of hydrogen, as further described below. It has been found that using stoichiometric excesses of hydrogen also improves the performance of two phase catalyst compositions which consist of hydrided titanium and the intermetallic compound having the approximate composition FeTi.

0042309

The invention for the first time permits the use of significantly lower operating temperatures and pressures in achieving commercially feasible production rates in the synthesis of alcohols. As the thermodynamics of the chemical system concerned permits higher equilibrium conversion at lower temperatures, the actial yields are greatly improved. Furthermore, again with respect to the alcohol synthesis, equipment requirements are much less stringent and less costly by reason of the lower operating pressures that can be employed at the process temperature selected. In addition, the use of the catalyst and the process of this invention greatly improves the economics of synthetic production of hydrocarbons and alcohols, which are valuable fuels and starting materials of many organic syntheses, by allowing the co-production of the same in an integral chemical complex which will produce the feed for the said synthesis through a more economical gasification process than heretofore possible, as well as employing a more economical process in the actual synthesis of hydrocarbons and alcohols.

The catalyst of this invention is free from many problems commonly encountered with prior art catalysts. For instance, there is little risk of carbonyl formation and also much less poisoning or deactivation of the catalyst through the smothering of adsorbing sites by the adsorption of impurities that normally exist in commercially available carbon monoxide. To the contrary, it has been found that the catalytic activity of the catalyst employed in the present invention increases with aging in an atmosphere containing hydrogen.

This enhancement of activity is the result of cracks and fissures formed in the catalyst particles, both microscopically (surface cracks) and macroscopically (breakage into smaller particles), with the attendant increase in the active surface area of the catalyst. Moreover, continuous cleaning of the catalyst surface from impurities by the hydrogen present in the reaction atmosphere prolongs the activity of the catalyst.

## Catalyst Activation Procedure

The two phase alloys used in preparing the preferred catalysts of the invention are usually received from the manufacturer in granular form. The granular material as received is coated with an oxide layer and in this state neither phase can form a hydride. Also the granules have other surface and internal impurities such as carbon and nitrogen compounds and adsorbed gases other than hydrogen and nitrogen. Initial cleaning of the particles and formation of the catalyst containing the titanium in hydrided form is desirably accomplished by charging the granular material as received into a suitable reactor and subjecting it to hydrogen gas, preferably at temperatures in the range of $200^{\circ}$C to $400^{\circ}$C. and at pressures in the range of 0 to 3000 p.s.i.g., preferably 150 to 200 p.s.i.g., for an extended period of time.

This initial exposure to hydrogen is followed by an outgassing step and then alternate pressurizing of the catalyst bed with hydrogen and outgassing so that phase B of the catalyst is successively hydrided and dehydrided. This process breaks up the catalyst granules into smaller particles and also produces multiple cracks in the surface of each individual granule, thereby greatly increasing the reactive surface area of the catalyst bed. The dehydriding step may be carried out at a temperature and hydrogen partial pressure that favors the decomposition of the hydride of phase B as determined by the thermodynamic equilibrium diagram of the inter-metallic compounds used. However, it is preferably carried out at or about $200^{\circ}$C. by drawing a slight vacuum of one or two inches of water on the reactor vessel containing the catalyst bed. Although the dehydriding may also be carried out by purging the reactor vessel with an inert gas such as helium, extreme care must be observed under such circumstances for the commercial grades of inert gases such as helium also contain oxygen at an impurity level which permanently poisons the catalyst by forming a stable oxide upon contact therewith at elevated temperatures.

The hydriding step may be carried out at a temperature and pressure that favors the formation of the hydride phase B as determined from the equilibrium conditions of the material used. Hydriding is preferably carried out at a temperature of $20^{o}$ to $25^{o}$C. and at a pressure of the order of 1,000 psia.

Any commercially available grade of hydrogen gas may be used in the activation process. However, the use of relatively high purity hydrogen permits the attainment of acceptable activation in a fewer number of cycles. Whether a less expensive grade of hydrogen or faster activation of the catalyst is desirable from the point of view of process economics, will depend upon the conditions at a given manufacturing plant.

If the hydrogen gas used in the activation process is less than 99.999% pure, then a high temperature hydrogen treatment step between the dehydriding and hydriding steps should be incorporated in the activation cycle. This added step is essentially the same as the initial hydrogen treatment and in this case removes the gases such as the impurities in the hydrogen gas used that become adsorbed on the catalyst particles during the hydriding step. Therefore, following the dehydriding step the reactor is filled with hydrogen gas at a temperature of about $200^{o}$ to $400^{o}$C. and a pressure of approximately 150 to 200 psia preferably for four to six hours. Following this high temperature hydrogen treatment the hydrogen gas is again evacuated from the reactor by drawing a vacuum and the catalyst bed is cooled to $20^{o}$ to $25^{o}$C. Upon reaching this temperature level, the reactor is pressurized with hydrogen gas at about 1000 psia and kept at these conditions until phase B is completely hydrided. The extent to which the hydriding reaction has proceeded may be followed by monitoring either temperature or pressure changes in the reactor and noting the time at which these parameters return to their initial values. Alternatively, the hydriding can be continued for a specific period of time, e.g., one-half hour.

After the hydriding reaction is completed the hydrogen is drawn off from the reactor by applying a vacuum until the pressure in the reactor is approximately one to two inches of water and concurrently the catalyst bed is heated to 200°C. in order to dehydride phase B. The foregoing hydriding-dehydriding cycle is carried out until phase B of the catalyst is capable of being hydrided and dehydrided very rapidly. In the case of catalyst compositions that are to be prepared in powdered form, the hydriding and dehydriding cycles are continued until the desired particle size is attained.

### Synthesis Process

The catalyst prepared as described above is charged to a suitable reactor and a gaseous feed stream comprised of hydrogen and carbon monoxide is continuously passed over the catalyst bed in the reactor at a suitable temperature and pressure. Higher temperatures favor the rate of production of the desired products. However, the temperatures that can be employed are limited by the equipment specification. Furthermore, there are thermodynamic restrictions on the temperatures that can be employed in the synthesis of alcohols which are formed at lower temperatures by equilibrium conditions. Although significant reaction rates are obtainable at temperatures as low as 80°C the practical temperature range appears to be about 200°C to 400°C.

Higher pressures favor both the rate of production of the desired products and the equilibrium concentrations of these products. The reaction may be carried out at a pressure within the range 1 to 200 atmospheres. The preferred range appears to be 30 to 100 atmospheres.

As indicated above, the feed stream for the process may be obtained by the gasification of coal, residua, oil shale or tar sands by any of the well known gasification techniques. The technology used will vary according to the nature of the material to be gasified. The gasification product is then subjected to a series of treatments to remove the components other than carbon monoxide and hydrogen, and the ratio of carbon monoxide to hydrogen adjusted to a desired level such as through water-gas shift reaction. This mixture of carbon monoxide and hydrogen is subsequently fed to the reactor that contains the catalyst either directly following the preceding step, or after having been mixed with the excess hydrogen that is present in the reaction medium and continuously recycled in the preferred embodiment.

As indicated above, in the preferred embodiment of the invention hydrogen is used in excess of the stoichiometric requirement and its proportion in the feed mixture can vary over a wide range, i.e. from a molar ratio of hydrogen to carbon monoxide of about 2:1 or 3:1, the stoichiometric ratios for production of methanol and methane, respectively, up to 33:1. Desirably the molar ratio of hydrogen to carbon monoxide is from 4:1 to 25:1 and preferably the ratio is from 10:1 to 20:1.

The invention is further described in the following examples.

## Example I

23.5 grams of a granular catalyst having the formula $Fe_{0.7}V_{0.2}Ti$ and having a particle size of 10-80 mesh and a surface area of 0.15 $m^2$/gram were charged to a 316 S.S. tubular reactor approximately 0.4" in inside diameter having a catalyst bed height of about 6". The catalyst was activated by subjecting it to five activation cycles. Each activation cycle included a high temperature step wherein the catalyst material was exposed to hydrogen at a temperature of 300-325$^{\circ}$C and a pressure of 200 psig for 6 to 12 hours and a low temperature step wherein the catalyst material was exposed to hydrogen at a temperature of 20$^{\circ}$C and at a pressure of 800 psig for less than one half hour.

After activation was complete, a gaseous mixture consisting of 4.78% by volume carbon monoxide and 95.22% hydrogen was passed continuously over the activated catalyst material. The synthesis reaction was conducted at a temperature of 300-310°C, a pressure of 380 psig and a flow rate of 67.34 SCCM (std cubic cm. per min).

The effluent gases first passed through a liquid trap wherein alcohols and higher hydrocarbons were condensed. The gases were analyzed by a Perkin Elmer Model 3920 gas chromatograph. The liquid was separately analyzed. Analysis indicated that 34.54% of the carbon monoxide in the feed was converted to products. The products had the following distribution.

|  | As Percent of Carbon Monoxide Converted | Relative Distribution of Gaseous Products |
|---|---|---|
| Methane | 56.32% | 93.60% |
| Ethane | 4.56% | 5.12% |
| Propane | 1.83% | 1.28% |
| Liquid ($C_{3+}$ and alcohols) | 37.29% | |

### Example II

20.9 grams of a granular catalyst having the formula $Fe_{0.7}Mo_{0.2}Ti_{1.0}$ and having a particle size of 10-80 mesh and a surface area of 0.15 $m^2$/gram were charged to the tubular reactor and activated as described in Example I.

After activation was complete, a gaseous mixture consisting of 4.7% by volume carbon monoxide and 95.3% hydrogen was passed continuously over the activated catalyst material. The synthesis reaction was conducted at a temperature of 300-305°C, a pressure of 360-370 psig and a feed rate of 50.74 SCCM.

The effluent gases were collected and analyzed as described in Example I.   Analysis indicated that 14.79% of the carbon monoxide in the feed was converted to products.   The products had the following distribution.

|  | As Percent of Carbon Monoxide Converted | Relative Distribution of Gaseous Products |
|---|---|---|
| Methane | 73.21% | 93.32% |
| Ethane | 8.52% | 5.42% |
| Propane | 3.19% | 1.36% |
| Liquid ($C_{3+}$ and alcohols) | 15.08% | |

## Example III

The catalyst of Example II was activated as described and used again with a feed mixture as described in Example II.

Analysis of the products indicated a 30.90% conversion of carbon monoxide to products and the products had the following distribution.

|  | As Percent of Carbon Monoxide Converted | Relative Distribution of Gaseous Products |
|---|---|---|
| Methane | 40.99% | 92.22% |
| Ethane | 5.28% | 5.94% |
| Propane | 2.46% | 1.85% |
| Liquid ($C_{3+}$ and alcohols) | 51.27% | |

- 13 -

0042309

## Example IV

21.9 grams of a granular catalyst having the formula $Fe_{1.0}Ti_{2.12}$ and having a particle size of 100-150 mesh and an estimated surface area of 0.05-0.50 $m^2$/gram were charged to the tubular reactor and activated as described in Example I.

After activation was complete, a gaseous mixture consisting of 4.79% by volume carbon monoxide and 95.21% hydrogen was passed continuously over the activated catalyst material. The synthesis reaction was conducted at a temperature of 250°C, a pressure of 380 psig and a feed rate of 44.5 SCCM.

The effluent gases were collected and analyzed as described in Example I. Analysis indicated that 23.2% of the carbon monoxide was converted to products. The products had the following distribution.

| | As Percent of Carbon Monoxide Converted | Relative Distribution of Gaseous Products |
|---|---|---|
| Methane | 35.74% | 79.04% |
| Ethane | 6.94% | 7.68% |
| Propane | 8.48% | 6.26% |
| Butane | 5.61% | 3.1% |
| Iso-butane | 0.23% | -- |
| Pentane | 4.70% | 2.08% |
| Hexane | 2.33% | 0.84% |
| Ethylene | 0.12% | 0.13% |
| Propylene | 0.93% | 0.69% |
| Butylene | -- | 0.13% |
| Liquid ($C_{7+}$ and alcohols) | 35.02% | -- |

Other useful embodiments and variations of the catalyst composition, the mode of preparation and treatment of the catalyst and synthesis steps will be apparent to those skilled in the art.

Other variations within the scope of the invention involve combining titanium dihydride with catalytically active metals for this reaction such as ruthenium and palladium either in the form of mixtures of multi-component (e.g. ternary, quaternary or higher) alloys, or supporting this catalyst upon inert carrier materials or other substrates.

By way of further example, activation of the catalyst can be achieved, although at a slower rate, by exposing the catalyst to hydrogen in the feed stream of the synthesis process itself, particularly, if the product stream is recycled until a desired product concentration is achieved.

A still further example of the variations in the process of the invention is carrying out the synthesis process by varying the ratio of carbon monoxide/hydrogen in the feed mixture in a cyclic manner as opposed to steadily feeding a given composition of the same.

The foregoing specification is not intended to be limited to the preferred embodiments of the invention described. Other embodiments are contemplated within the scope of the appended claims.

- 15 -                                                          0042309

WHAT IS CLAIMED IS:

1.    A method of making hydrocarbons and alcohols which comprises contacting carbon monoxide and hydrogen in synthesis proportions and under synthesis conditions over a catalyst consisting essentially of an intermetallic compound having a first and second phase,

said first phase being selected from:

(1) a single metal selected from V and Nb,

(2) a single phase binary alloy selected from TiCo, TiCr, TiCu and TiMn and the binary Laves phase compounds $TiCr_2$, $ZrNi_2$, $ZrMo_2$, $ZrFe_2$, $ZrCo_2$, $ZrMn_2$, $TiCo_2$, $ZrCu_2$ and $ZrCr_2$, and

(3) single phase ternary or quaternary alloys selected from

(a) $TiFe_x M_y N_z$ where M and N are any metal that can substitute for Fe such that the resulting alloy is a single phase alloy, but not Ni or Ru, and where X is a number less than 1.0, y and z are each numbers less than 1.0 or either, but not both, may be 0, and x and y and z is approximately 1.0;

(b) $TiA_x M_y N_z$ where A is Co, Cr, Mn or Cu, M and N are any metal that can substitute for Co, Cr, Mn or Cu such that the resulting alloy is a single phase alloy, and where x is a number less than 1.0, y and z are each numbers less than 1.0 or either, but not both, may be 0, and x and y and z is approximately 1.0; and

(c) ternary or higher Laves phase compounds based on Ti and/or Zr, and

said second phase is free titanium hydride or zirconium hydride,
and thereby forming hydrocarbons and alcohols.

2.    A method as claimed in claim 1 wherein the catalyst is activated prior to the synthesis reaction, the free titanium hydride or zirconium hydride being formed under the conditions of catalyst activation and being maintained under the conditions of the synthesis process.

3.    A method as claimed in claim 1 wherein the ternary or higher Laves phase compounds are:

(i) $TiB_x M_y N_z$ where B is Cr or Co, M and N are any metals that can substitute for Cr or Co such that the resulting alloy is a single phase alloy, and where x is a number less than 2.0, y and z are each numbers less than 2.0 or either, but not both, may be 0, and x and y and z is approximately 2.0 and

(ii) $ZrC_x M_y N_z$ where C is Ni, Mo, Fe, Co, Mn, Cr or Cu, M and N are any metals that can substitute for Ni, Mo, Fe, Co, Mn, Cr or Cu such that the resulting alloy is a single phase alloy, and where x is a number less than 2.0, y and z are each numbers less than 2.0 or either, but not both, may be 0, and x and y and z is approximately 2.0.

4. A method as claimed in any of claims 1 to 3 wherein the first phase is a ternary or quaternary alloy of the formula $TiFe_x M_y N_z$ as defined above where M and N are Co, Cr, Mn, Mo, V and Cu.

5. A method as claimed in any preceding claim wherein the second phase is $TiH_{1.98}$.

6. A method as claimed in any preceding claim wherein the molar ratio of hydrogen to carbon monoxide is 2:1 to 33:1.

7. A method as claimed in any preceding claim wherein the synthesis is carried out in the presence of a stoichiometric excess of hydrogen, the molar ratio of hydrogen to carbon monoxide being 10:1 to 20:1.

8. A method of making hydrocarbons and alcohols which comprises contacting carbon monoxide and hydrogen under synthesis conditions over a catalyst consisting essentially of hydrided titanium and the intermetallic compound having the approximate composition FeTi in the presence of a stoichiometric excess of hydrogen, the molar ratio of hydrogen to carbon monoxide being from 2:1 to 33:1.

9. A method as claimed in claim 8 wherein the molar ratio of hydrogen to carbon monoxide is 10:1 to 20:1.

10.  A method as claimed in claim 1 where the catalyst is as defined in subparagraph (3)(a) and wherein M is V and z is 0.

11.  A method as claimed in claim 1 wherein the catalyst is as defined in subparagraph (3)(a) and wherein M is Mo and z is 0.

12.  A method of making hydrocarbons and alcohols which comprises contacting carbon monoxide and hydrogen in synthesis proportions and under synthesis conditions over a catalyst consisting essentially of an intermetallic compound having a first and second phase,

said first phase being selected from:

(1) a single metal selected from V and Nb,

(2) a single phase binary alloy selected from TiCo, TiCr, TiCu and TiMn and the binary Laves phase compounds $TiCr_2$, $ZrNi_2$, $ZrMo_2$, $ZrFe_2$, $ZrCo_2$, $ZrMn_2$; $TiCo_2$, $ZrCu_2$ and $ZrCr_2$, and

(3) single phase ternary or quaternary alloys selected from

(a) $TiFe_xM_yN_z$ where M and N are any metal that can substitute for Fe such that the resulting alloy is a single phase alloy, but not Ni or Ru, and where X is a number less than 1.0, y and z are each numbers less than 1.0 or either, but not both, may be 0, and x and y and z is approximately 1.0;

(b) $TiA_xM_yN_z$ where A is Co, Cr, Mn or Cu, M and N are any metal that can substitute for Co, Cr, Mn or Cu such that the resulting alloy is a single phase alloy, and where x is a number less than 1.0, y and z are each numbers less than 1.0 or either, but not both, may be 0, and x and y and z is approximately 1.0; and

(c) ternary or higher Laves phase compounds based on Ti and/or Zr, and

said second phase is free titanium hydride or zirconium hydride,

said catalyst having been activated prior to the synthesis reaction to form free titanium hydride or zirconium hydride, and thereby forming hydrocarbons and alcohols.

13.  A method as claimed in claim 12 wherein the free titanium hydride or zirconium hydride is maintained under the
conditions of the synthesis process.

## DOCUMENTS CONSIDERED TO BE RELEVANT

| Category | Citation of document with indication, where appropriate, of relevant passages | Relevant to claim |
|---|---|---|
| P | CHEMICAL ABSTRACTS, vol. 94, no. 20, May 18, 1981 page 150, abstract 159338d Columbus, Ohio, USA LAPIDUS, A.L. "Catalytic activity of intermetallic hydrides in the hydrogenation of carbon monoxide" & IZV. AKAD.NAUK SSSR, SER.KHIM. 1980, (11), 2452-5. * Whole abstract * | 1,12 |
| A | CHEMICAL ABSTRACTS, vol. 81, no. 23, December 9, 1974 page 467, abstract 151363u Columbus, Ohio, USA POLYAKIN Yu,L. et al. "Nickel-zirconium catalyst for the hydrogenation of carbon monoxide to higher hydrocarbons" & FIZ-KHIM. METODY ANCL. KONTROLYA PROIZVOD. 1973, 13-16 | |
| AD | US - A - 4 139 551 (OZYAGCILAR) | |
| E | EP - A - 0 035 328 (M.N. OZYAGCILAR) * Pages 10-11, 14-16; claims * | 1,3, 5-13 |

**CLASSIFICATION OF THE APPLICATION (Int. Cl.³)**

C 07 C 1/04
29/15
B 01 K 31/12

**TECHNICAL FIELDS SEARCHED (Int. Cl.³)**

C 07 C 1/04
1/02
29/15
B 01 J 31/38
31/36
31/34
31/32
31/28
31/26
31/12

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 16-09-1981 | VAN GEYT |

EPO Form 1503.1 06.78